# EUROPEAN PATENT APPLICATION

(11) **EP 4 461 286 A1**
(43) Date of publication of application: **13.11.2024**
(21) Application number: 23172549.0
(22) Date of filing: 10.05.2023
(51) Int. Cl.: A61K 6/62, A61K 6/887, B29C 64/40, B33Y 10/00, B33Y 70/00

(54) **A DENTAL SYSTEM COMPRISING AT LEAST ONE DENTAL SUPPORT ELEMENT AND A 3-DIMENSIONAL PRINTING PROCESS FOR MANUFACTURING THEREOF**

(71) Applicant: DENTSPLY SIRONA Inc., York, PA 17401 (US); DENTSPLY DETREY GmbH, 78467 Konstanz (DE)
(72) Inventor: Tigges, Thomas, 78567 Konstanz (DE); Scheufler, Christian, 78567 Konstanz (DE); Xiaoming, Jin, 78567 Konstanz (DE); Huo, Xin, Delaware (US); Liu, Yi, Delaware (US); Weber, Christoph, 78764 Konstanz (DE)
(74) Representative: Pichova, Vanda

(57) **Abstract**

The invention discloses a dental system comprising at least one dental support element and at least one dental restoration element, wherein the at least one dental support element comprises at least one dental support material comprising a photocurable polymerizable monomer having at least one (meth)acrylate group and wherein the at least one dental restoration element comprises at least one dental restoration material comprising a photocurable polymerizable monomer having at least one (meth)acrylate group. The dental support material may comprise a thermo reversible hydrogel composition comprising at least one first and at least one second polysaccharide being different to each other. The invention also discloses a 3-dimensional printing process for the manufacturing the dental system.

## Description

### BACKGROUND OF THE INVENTION

The present invention relates to a dental system comprising at least one dental support element and at least one dental restoration element and to a 3-dimensional printing process for manufacturing thereof.

For most of the 3D printing applications, a support structure is mandatory to ensure dimensional accuracy of the printed part. In case of extrusion -based 3D-printing (Paste extrusion modeling, PEM), the support structure can be printed from a different material than the actual printed part. This allows for facile removability of the support structure while lowering the risk of damaging the printed part.

### The following terms and abbreviations are used in the present patent specification :

3D = 3-dimensional
PEM = Paste extrusion modeling
PEGMA = Polyethyleneglycol dimethacrylate
PDMS = Polydimethylsiloxane
TMDI = Trimethyl-hexamethylene diisocyanate
Igepal = Octylphenoxypolyethoxyethanol (nonpolymerizable detergent )
BAPO = Phenyl-bis -(2,4,6- trimethylbenzoyl)-phosphinoxide
DMABN = 4-(N,N-dimethylamino)benzonitrile
DPI = Dimethyl diphenyl iodonium hexafluorophosphate
Aerosil 200 = a hydrophilic fumed silica
Aerosil VPR 711 = a surface modified fumed silica
LBG = Locust Bean Gum
DEGDVE = diethyleneglycol divinylether
TEGDVE = triethyleneglycol divinylether
Dental restoration element = 3D dental restoration element
LED = a light-emitting diode
E163 = E 163 is an additive approved by the European Union (EU). It is used as a natural colouring agent
Resin A= a reaction product of TMDI, polypropylene glycol diol and polyethylene glycol methacrylate
Resin B= a reaction product of hydroxyl terminated Siloxane, TMDI, polypropylene glycol diol and polyethylene glycol methacrylate

The prior art document WO 2019/222599 discloses a light-curable dental impression material comprising: (a) a polymerizable polysiloxane resin composition comprising compounds of the following formula (I): E-(L¹-Z)ₙ- L²-E, wherein the E which may be the same or different, independently represent a monovalent group selected from a group containing a polymerizable carbon-carbon double bond, a group containing a polysiloxane moiety, a C₂₋₂₀ alkoxy group, a C₂₋₂₀ thioalkyl group, and a RNH group, wherein R is a C₂₋₂₀ alkyl group; L¹ which may be the same or different when more than one L¹ is present, represents a divalent group of the following formula (II): wherein L³ which may be the same or different when more than one L³ is present, independently represents a divalent linker group which may be substituted with up to four substituents of the formula (III): -L¹-E, wherein L¹ and E are as defined above; L⁴ which may be the same or different when more than one L⁴ is present, independently represents a divalent linker group which may be substituted with up to four substituents of the formula (III), wherein L¹ and E are as defined above; X¹, X², Y¹ and Y², which may be the same or different, and when more than one X¹, X², Y¹ or Y², is present, the X¹, X², Y¹ and Y² may be the same or different, independently represent an oxygen atom, a sulfur atom and a group NR', wherein R' is a hydrogen atom or a C₁₋₄ alkyl group; m represents 0 or an integer of from 1 to 40; Z represents a divalent linker group which may additionally be substituted with up to four substituents selected from polysiloxane groups and groups of the formula (III), wherein L¹ and E are as defined above;L² represents a single bond or a divalent group of the formula (II), wherein L³, L⁴, X¹, X², Y¹, Y² and m are independently as defined for L¹; n represents 0 or an integer of from 1 to 4; provided that a compound of formula (I) contains at least one monovalent group E having a polymerizable carbon-carbon double bond and a compound of formula (I) contains at least one polysiloxane group, and provided that when n is 0, then L² is a divalent group of the formula (II); (b) a particulate filler; and (c) a photoinitiator. The dental impression material is highly tolerant to moisture, has adjustable working and setting times, has excellent tear resistance while providing at the same time good detail reproduction without adhesion to core build-up materials or composite restorations, excellent dimensional stability, reduced shrinkage onset and may be provided as a single composition which does not need mixing prior to use.

### DETAILED DESCRIPTION OF THE INVENTION

It is an object of the present invention to provide a dental system comprising at least one dental support element and at least one dental restoration element and a 3-dimensional printing process for manufacturing thereof.

The present invention discloses a dental system comprising materials which provide both support for the at least one dental restoration element and also an ease of its removability from the at least one dental support element, preferably in less than 5 minutes, without negatively influencing the quality of the at least one dental restoration element, which is based on a specific material formulation and/or selective light curing by using LED with distinct and narrow emission spectra. A printed at least one dental restoration element has a sufficient shelf-life and storage stability, preferably of more than 6 months.

One of the advantages of the dental system of the present invention is that the dental system is a one-part system and as a result of that no mixing is required before the printing.

Preferably, the material of the at least one dental support element is flexible after final curing.

Preferably, the material of the at least one dental support element has low adhesion to the material of the at least one restoration element after final curing.

Preferably, a non-cured material of the at least one dental support element has a slump resistance to facilitate dimensional accuracy during printing.

The material of the at least one dental support element can be heated to reduce pressure needed for extrusion.

Preferably, the material of the at least one dental support element can be heated to 65 °C for a prolonged period of time without significantly changing its properties.

The present invention discloses a dental system comprising at least one dental support element and at least one dental restoration element, wherein the at least one dental support element comprises at least one dental support material comprising a photocurable polymerizable monomer having at least one (meth)acrylate group and wherein the at least one dental restoration element comprises at least one dental restoration material comprising a photocurable polymerizable monomer having at least one (meth)acrylate group.

In the preferred embodiment of the present invention, the weight ratio of the photocurable polymerizable monomer having at least one (meth)acrylate group in the at least one dental support material to the photocurable polymerizable monomer having at least one (meth)acrylate group in the at least one dental restoration material is 1: 10 - 1:500, preferably 1:20 to 1:250 and most preferably 1:200.

In the preferred embodiment of the present invention, the at least one dental support material comprises 0.1-3.0 weight % based on the total weight of the at least one dental support material of the photocurable polymerizable monomer having at least one (meth)acrylate group.

The photocurable polymerizable monomer having at least one (meth) acrylate group is preferably selected from the group comprising a monofunctional or polyfunctional (meth)acrylate including but not limited to methyl (meth)acrylate, ethyl (meth)acrylate, propyl (meth)acrylate, isopropyl(meth)acrylate, 2-hydroxyethyl(meth)acrylate, hydroxypropyl (meth)acrylate, tetrahydrofurfuryl(meth)acrylate, glycidyl(meth)acrylate, the diglycidyl (meth)acrylate of bis-phenol A, glycerol mono- and di- (meth)acrylate, ethyleneglycol di(meth)acrylate, polyethyleneglycol di(meth)acrylate (where the number of repeating ethylene oxide units vary from 2 to 30 especially triethylene glycol di(meth)acrylate), neopentylglycol di(meth(acrylate, trimethylol propane tri(meth)acrylate, mono-, di-, tri-, and tetra- acrylates and (meth)acrylates of pentaerythritol and mono-, di-, tri-, tetra-, penta- and hexa- acrylates and (meth)acrylates of dipentaerythritol, 1,3-butanedioldi(meth)acrylate and 1,4-butanediol di(meth)acrylate.

In another aspect of the present invention, the at least one dental support material and the at least one dental restoration material each comprises a photoinitiator composition, wherein the two photoinitiator compositions are identical.

In another aspect of the present invention, the two photoinitiator compositions are not identical. In the preferred embodiment of the present invention, the absorption maxima of the two photoinitiator compositions differ by at least 50nm.

The irradiation devices for 3D printing often consist of light sources with different emissions. Various advantages are associated with this, such as gradual curing of the materials to be polymerized or different depths of polymerization.

In the preferred embodiment of the present invention, the photoinitiator composition comprises at least one compound selected from the group comprising Camphorquinone, DMABN, DPI, BAPO and Diphenyl (2,4,6-(trimethylbenzoyl)-phosphinoxide. When aiming at selective light curing, a photoinitiator composition comprising Camphorquinone, DMABN and DPI is used for initiation at 470nm, while a photoinitiator composition comprising Phenyl-bis-(2,4,6- trimethylbenzoyl)-phosphinoxie (BAPO) and/or Diphenyl (2,4,6-(trimethylbenzoyl)-phosphinoxide is be used for curing at 365 nm.

In the preferred embodiment of the present invention, the dental system comprises 0.5- 3.0 weight % of the total of the two photoinitiator compositions based on the total weight of the dental system.

In another aspect of the present invention, the at least one dental support material comprises 3.5-50 weight %, more preferably 15-25 weight %, even more preferably 21 weight % , based on the total weight of the at least one dental support material, of at least one copolymerizable monomer copolymerizable with (meth) acrylate groups that is not a (meth)acrylate itself, wherein the copolymerizable monomer has preferably at least one of polymerizable groups comprising vinyl (excluding meth (acrylates)) and allyl.

The at least one copolymerizable monomer is preferably selected from the group comprising vinyl ethers.

In the preferred embodiment of the present invention, the at least one copolymerizable monomer is selected from the group comprising tetra (ethylene glycol) divinyl ether, diethyleneglycol divinylether (DEGDVE) and triethyleneglycol divinylether (TEGDVE).

The at least one dental support material may also comprise a nonpolymerizable surfactant, preferably Igepal 630 (Octylphenoxypolyethoxyethanol). The at least one dental support material comprises preferably 0.5-2.0 weight % of the nonpolymerizable surfactant.

The at least one dental support material may also comprise at least one filler, including surface modified fumed silica, preferably Aerosil VPR 711. In the preferred embodiment of the present invention, the at least one dental support material comprises 10-40 weight % of the at least one filler, based on the total weight of the at least one support material.

In another aspect of the present invention, the at least one dental support material comprises Resin B, selected from the group comprising a reaction product of hydroxyl terminated Siloxane, TMDI, polypropylene glycol diol and polyethylene glycol methacrylate. Preferably, the content of the Resin B in the at least one dental support material is 5.0-70 weight %, based on the total weight of the at least dental support material. The preferred composition of the at least one dental support material is depicted in Table 1. The composition of the Table 1 has optimized properties in respect of release of the at least one dental support element from the at least one dental restoration element.

**Tab. 1: Composition of the at least on dental support material**

| **Compound** | **Category** | **Content /weight %** |
|---|---|---|
| Tetra (ethylene glycol) divinyl ether | A copolymerizable monomer | 21.0 |
| (Polypropylene glycol - PDMS - TMDI-PEGMA) copolymer | Resin B | 63.3 |
| Trimethylpropane tri(meth)acrylate | A photocurable polymerizable monomer | 1.0 |
| Aerosil VPR 711 | A surface modified fumed silica | 12.7 |
| Phenyl-bis-(2,4,6-trimethylbenzoyl)-phosphinoxide (BAPO) | A photoinitiator | 1.0 |
| Igepal 630 | A nonpolymerizable surfactant | 1.0 |
| **Total** | | **100** |

In another aspect of the present invention, the at least one dental support material comprises Resin A, selected from the group comprising a reaction product of TMDI, polypropylene glycol diol and polyethylene glycol methacrylate.

A composition of a dental support material with improved tear strength is depicted in Table 2.

**Tab. 2: Composition of a dental support material with improved tear strength**

| Compound | Category | Content/weight % |
|---|---|---|
| (Polypropylene glycol-TMDI-PEGMA) copolymer | Resin A | 80.1 |
| (Polypropylene glycol - PDMS - TMDI-PEGMA) copolymer | Resin B | 8.9 |
| Aerosil VPR 711 | A surface modified fumed silica | 10.0 |
| Phenyl-bis-(2,4,6-trimethylbenzoyl)-phosphinoxide (BAPO) | A photoinitiator | 1.0 |
| Total | | 100 |

Another of the advantages of the dental system of the present invention is that the at least one dental support material of the at least one dental support element is curable by irradiation; it is selectively curable by light of a given wavelength while having little effect on the at least one dental restoration material of the at least one restoration element.

For selective curing it is important that irradiation with different wavelength is conducted sequentially.

Preferably, the at least one dental support material of the at least one dental support element does not leave any residues on the at least one dental restoration element and the removal of the at least one dental restoration element is possible without excessive mechanical force or use of the solvent. It is important that the material of the at least one dental support element has a high tear strength to facilitate removability without rupture after final curing.

In another aspect of the present invention, the at least one dental support material comprises a thermo reversible hydrogel composition comprising at least one first and at least one second polysaccharide being different to each other, wherein the thermo reversible hydrogel composition comprises from 0.5 to 5.0 weight % based on the total weight of the at least one dental support material of each of the at least one first and at the least one second polysaccharide.

In another aspect of the present invention, the thermo reversible hydrogel composition comprises at least one first polysaccharide selected from the group comprising Xanthan, Psyllium husk, Chia seeds, ground flax, cornstarch, unflavored gelatin, egg whites, Agar Agar and Guar Gum and at least one second polysaccharide selected from the group comprising galactomannans, including Locust Bean Gum, wherein the weight ratio of the at least one first polysaccharide to the at least one second polysaccharide is of 20:1 to 1:20, preferably 15:1 to 1:15, more preferably 12:1 to 1:12 and most preferably 1:1. The strength of the resulting thermo reversible hydrogel composition can be tuned by varying the ratio between the at least one first and the at least one second polysaccharide. The strongest thermo reversible hydrogel composition is obtained when the ratio is close to 1:1 by weight of the at least one first polysaccharide and the at least one second polysaccharide.

The thermo reversible hydrogel composition may also comprise a hydrophilic fumed silica, selected from the group comprising Aerosil 200, Aerosil 255 and Aerosil 380. The preferred content of the hydrophilic fumed silica is 2-10 weight % based on the total weight of the thermo reversible hydrogel composition. The hydrophilic fumed silica is added to increase the viscosity of the at least one dental support material in the molten state, allowing better control during extrusion and a sufficient slump resistance during 3D-printing.

In the preferred embodiment of the present invention, the thermo reversible hydrogel composition comprises Xanthan and Locust Bean Gum. In the preferred embodiment, the thermo reversible hydrogel composition comprises the following ingredients, preferably in respective weight %: water (92.0), Xanthan (1.5), Locust Bean Gum (1.5) and Aerosil 200 (5.0).

A strong mismatch in the weight % of the at least first and the at least second polysaccharides may result in a weak thermo reversible hydrogel composition or even a viscous liquid.

The thermo reversible hydrogel composition provides sufficient strength to be used as a separation layer between the at least one dental support element and the at least one dental restoration element.

The thermo reversible hydrogel composition may comprise a pigment or a coloring agent. Suitable coloring agents are those which can be dispersed or dissolved in water, including all materials from the E163 group.

The thermo reversible hydrogel composition may comprise a biocide including sodium cyanide or a preservative including sorbic acid (E200).

The thermo reversible hydrogel composition has preferably a melting point ranging from 50 to 70⁰C.

The thermo reversible hydrogel composition does not show any adhesion to any of the materials of the at least one dental support element or the at least one dental restoration element. After printing, the thermo reversible hydrogel composition can be rapidly separated from the at least one restoration element and/or from the at least one dental support element by use of light mechanical force or by rinsing with water.

The thermo reversible hydrogel composition can be used in combination with the at least one material of the at least one dental support element and/or with the at least one material of the at least one dental restoration element.

The thermo reversible hydrogel composition acts as a 3D-printable release agent.

The present invention discloses a 3-dimensional printing process for manufacturing the dental system described above.

The 3-dimensional printing process according to the present invention is an extrusion based 3-dimensional printing process, wherein layers of the at least one dental support material and the at least one dental restoration material are applied for the manufacturing of the dental system.

The 3-dimensional printing process comprises preferably the following process steps in the following order:
a) providing the at least one dental support material as described above
b) providing the at least one dental restoration material as described above
c) extruding at least one layer for the manufacturing of the dental system comprising the at least one dental support material for the respective at least one dental support element parts inside of each of these layers, and the at least one dental restoration material for the respective at least one dental restoration element parts inside of each of these layers to produce at least two extruded layers and
d) photocuring the at least one extruded layer of process step c) by a light source.

The process step d) is preferably executed in such a way that first the respective at least one dental support element parts inside of each of the at least one extruded layer are photocured by a light source, and then subsequently the respective at least one dental restoration element parts inside of each of these at least one extruded layer are photocured by a light source.

In another aspect of the present invention, the at least one dental support material is for use in manufacturing the at least one dental support element which is releasable from the at least one dental restoration element comprising the at least one dental restoration material to an extent of at least 90 %, preferably at least 95 %, more preferably at least 99 % based on the total surface of the at least one dental restoration element, wherein the at least one dental support element and the at least one dental restoration element are both manufactured by a 3-dimensional printing process, wherein the at least one dental support material comprises a thermo reversible hydrogel composition having a melting point ranging from 50 to 70⁰C, the thermo reversible hydrogel composition comprising at least one first and a at least one second polysaccharide being different to each other.

The at least one dental support material for use as described above, wherein one of the at least one dental support element forms an intermediate layer between the at least one dental restoration element and another of the at least one dental support element, with the proviso that the at least one dental support element is at least two dental support elements.

## Claims

1. A dental system comprising at least one dental support element and at least one dental restoration element, wherein the at least one dental support element comprises at least one dental support material comprising a photocurable polymerizable monomer having at least one (meth)acrylate group and wherein the at least one dental restoration element comprises at least one dental restoration material comprising a photocurable polymerizable monomer having at least one (meth)acrylate group, wherein the weight ratio of the photocurable polymerizable monomer having at least one (meth)acrylate group in the at least one dental support material to the photocurable polymerizable monomer having at least one (meth)acrylate group in the at least one dental restoration material is 1:10 - 1:500.

2. The dental system according to claim 1, wherein the weight ratio of the photocurable polymerizable monomer having at least one (meth)acrylate group in the at least one dental support material to the photocurable polymerizable monomer having at least one (meth)acrylate group in the at least one dental restoration material is 1:200.

3. The dental system according to claims 1 -2, wherein the at least one dental support material and the at least one dental restoration material each comprises a photoinitiator composition, wherein the two photoinitiator compositions are identical.

4. The dental system according to claims 1-3, wherein the at least one dental support material and the at least one dental restoration material each further comprises a photoinitiator composition, wherein the two photoinitiator compositions are not identical.

5. The dental system according to claim 4, wherein the absorption maxima of the two photoinitiator compositions differ by at least 50 nm.

6. The dental system according to any of the preceding claims, wherein the at least one dental support material comprises 3.5-50 weight %, based on the total weight of the at least one dental support material of at least one copolymerizable monomer copolymerizable with (meth)acrylate groups that is not a (meth)acrylate itself.

7. The dental system according to claim 6, wherein the copolymerizable monomer comprises at least one of polymerizable groups comprising vinyl, excluding meth(acrylates) and allyl.

8. The dental system according to any of the preceding claims, wherein the at least one dental support material comprises Resin B.

9. The dental system according to any of the preceding claims, wherein the at least one dental support material comprises a thermo reversible hydrogel composition having a melting point ranging from 50 to 70°C.

10. The dental system according to claim 9, wherein the thermo reversible hydrogel composition comprises at least one first and at least one second polysaccharide being different to each other, wherein the thermo reversible hydrogel composition comprises from 0.5 to 5.0 weight % based on the total weight of the at least one dental support material of each of the at least one first and the at least one second polysaccharide.

11. The dental system according to claim 10, wherein the thermo reversible hydrogel composition comprises the at least one first polysaccharide in a weight ratio to the at least one second polysaccharide of 1:1.

12. A 3-dimensional printing process for the manufacturing of the dental system according to claims 1-12, wherein the process is an extrusion based 3-dimensional printing process, wherein layers of the at least one dental support material and the at least one dental restoration material are applied for the manufacturing of the dental system, wherein the process comprises the following process steps in the following order:
a) providing the at least one dental support material as described above
b) providing the at least one dental restoration material as described above
c) extruding at least one layer for the manufacturing of the dental system comprising the at least one dental support material for the respective at least one dental support element parts inside of each of these layers, and the at least one dental restoration material for the respective at least one dental restoration element parts inside of each of these layers to produce at least one extruded layer and
d) photocuring the at least one extruded layers of process step c) by a light source.

13. The process of claim 12, wherein the process step d) is executed in such a way that first the respective at least one dental support element parts inside of each of the at least one extruded layer are photocured by a light source, and then subsequently the respective at least one dental restoration element parts inside of each of these at least one extruded layer are photocured by a light source.
